# EUROPEAN PATENT SPECIFICATION

(11) **EP 3 155 991 B1**
(45) Date of publication and mention of the grant of the patent: **15.01.2020**
(21) Application number: 15189811.1
(22) Date of filing: 14.10.2015
(51) Int. Cl.: A61B 18/14, A61B 18/00, A61B 17/00, A61B 17/29

(54) **DISTAL HEAD ASSEMBLY FOR A BIPOLAR INSTRUMENT, BIPOLAR INSTRUMENT AND METHOD OF ASSEMBLING A DISTAL HEAD ASSEMBLY**
DISTALE KOPFANORDNUNG FÜR EIN BIPOLARES INSTRUMENT, BIPOLARES INSTRUMENT UND VERFAHREN ZUR MONTAGE EINER DISTALEN KOPFANORDNUNG
ASSEMBLAGE TÊTE DISTALE POUR UN INSTRUMENT BIPOLAIRE, INSTRUMENT BIPOLAIRE ET PROCÉDÉ D'ASSEMBLAGE D'UN ENSEMBLE DE TÊTE DISTAL

(43) Date of publication of application: 19.04.2017
(73) Proprietor: Karl Storz SE & Co. KG, 78532 Tuttlingen (DE)
(72) Inventor: Thouément, Yann, 78690 Les Essarts le Roi (FR); Besse, Régis, 78610 Le Perray-en-Yvelines (FR)
(74) Representative: Witte, Weller & Partner Patentanwälte mbB

(56) References cited:
- WO-A1-2015/088655
- US-A1- 2006 074 415
- US-A1- 2012 053 405

## Description

The present disclosure relates to a distal head assembly for a bipolar instrument, and to a bipolar instrument that is fitted with a respective head assembly, particularly a bipolar coagulating instrument selected from the group consisting of bipolar forceps, bipolar scissors, a bipolar clamp, and bipolar pincers. The present disclosure further relates to a method of assembling a distal head assembly for a bipolar instrument.

Bipolar instruments may generally involve electrosurgical forceps. Bipolar instruments may be arranged for open surgery, but also for minimal-invasive surgery. To this end, endoscopic or laparoscopic bipolar instruments are known that comprise an elongated shaft defining a distal end and a proximal end, the distal end thereof being arranged for insertion into the human or animal body.

A similar bipolar electrosurgical instrument is known from US 8,361,071 B2, the instrument comprising first and second end effectors, and first and second handle members disposed proximal of the end effectors, wherein at least one of the handle members is movable from a first position to a second position, wherein the end effectors are spaced from one another in the first position and arranged in close proximity in the second position, the instrument further comprising a first electrode disposed on the first end effector and a second electrode disposed on the second end effector, the first and second electrodes facing one another and respectively comprising a tissue contacting surface to engage tissue between the first and second end effectors for applying electrosurgical energy to the tissue grasped therebetween to effect sealing. Bipolar instruments are commonly known in the field of surgery. Bipolar forceps utilize both mechanical clamping action and electrical energy for tissue treatment and/or treatment of blood vessels.

US 2006/074415 A1 discloses a electrosurgical end effector cartridge arranged to couple to a robotic surgical instrument of a robotic surgical system, the electrosurgical end effector cartridge comprising a first moveable end effector having a jaw portion and a base portion, a first spring latch coupled to the first moveable end effector near the base portion and arranged to couple to a first receptacle of a robotic surgical instrument, and a second moveable end effector having a jaw portion and a base portion, wherein the jaw portion of the first moveable end effector and the jaw portion of the second moveable end effector are arranged to couple to tissue in a surgical site.

Bipolar instruments, particular bipolar forceps, generally utilize two electrodes which are basically arranged in opposite fashion, wherein the electrodes are arranged on opposing surfaces of jaw-like end effectors, and wherein the electrodes are coupled to an electrosurgical generator. Human or animal tissue is in general a conductor of electrical energy. This may be utilized by clamping or grasping a respective tissue portion between the electrodes of the instrument, wherein the electrodes are charged to a different electric potential, such that electrical energy is transferred through the conducting tissue.

Hence, coagulation or hemostasis may be effected. In other words, bleeding may be stopped, reduced or at least slowed. Further, some bipolar instruments are known that may be utilized to not only clamp but cut tissue.

Particularly for minimal-invasive surgery applications, there is a need to provide minimized bipolar instruments, particularly minimized distal heads for such instruments. Preferably, the distal head of such an instrument has an overall diameter, at least in an insertion state, that is not larger or not essentially larger than the diameter of the shaft of the instrument. This poses several challenges to the design of such distal ends, given that typically a movable jaw arrangement is provided which needs to be contacted by electrical leads and which further needs to be contacted by a driving mechanism to effect an opening and closing movement of the jaw arrangement.

Another issue is that the jaws of the jaw arrangement further define opposing electrodes which are, when in operation, at least temporarily chargeed to a different electric potential. Therefore, isolating the jaws, particularly the live (current-carrying) portions thereof is crucial for proper operation and performance. A further challenge may arise in connection with assembly operations since arranging and mounting minimized parts often requires elaborate assembly steps.

It is therefore an object of the present invention to provide an improved distal head assembly for a bipolar instrument, a related assembly method, and a respective bipolar instrument that is fitted with the head assembly, wherein the head assembly is easy to manufacture and assemble, and wherein at least some of the above-indicated drawbacks are addressed. Further, a bipolar instrument and a distal head assembly therefor shall be provided which preferably exhibit an improved operation performance and reliability. It is further preferred to present a bipolar instrument and a distal head arranged for being attached thereto which are less prone to short circuits at the distal head. The invention is defined in the appended independent claims 1 and 14. Preferred embodiments are disclosed in the dependent claims.

In accordance with a first aspect of the present disclosure, a distal head assembly for a bipolar instrument is presented, the head assembly comprising a receiving frame comprising a first lateral arm and a second lateral arm that define therebetween a receiving portion, a transmitting piece movably arranged at the receiving portion, a movable jaw arrangement comprising a first jaw and a second jaw, and a retaining bracket arranged to engage the receiving frame, wherein the jaw arrangement is pivotably mounted to the receiving frame in a snap-on mounting fashion, wherein the retaining bracket, in the mounted state, engages the receiving frame and secures the mounted state of the jaw arrangement, and wherein the retaining bracket is arranged as a frontal locking bracket that is arranged to be attached to a frontal end of the receiving frame to secure the snap-on mounting of the jaw arrangement.

This aspect is based on the insight that manufacturing and assembling the distal head may be greatly simplified by providing a snap-on or clip-on interface between the receiving frame and the components of the distal head that are mounted thereto. This may particularly involve that the receiving frame is arranged in a fashion at least partially deflectable. Hence, by deflecting the receiving frame, an assembly space is provided which is sufficiently large to allow the transmitting piece and the jaw arrangement to approach their mounting position. Once the transmitting piece and the movable jaw arrangement are adequately positioned, the receiving frame may be relieved (flexed back) which may involve an engagement of respective co-operating mounting elements or the receiving frame, the transmitting piece and the movable jaw arrangement, respectively. Consequently, the transmitting piece and the movable jaw arrangement may be attached to the receiving frame in a positive-locking fashion. The retaining bracket secures the mounted state and particularly prevents the receiving frame from being deflected again. Therefore, the positive-fit arrangement and also the overall shape of the receiving frame is maintained.

The receiving frame may also be referred to as receiving fork, whereas the first lateral arm and the second lateral arm may be referred to as first and second fork arms. The receiving portion may be referred to as receiving space or gap between the fork arms. The receiving frame may be arranged to be attached to a basically longitudinally extending shaft of the bipolar instrument. In some embodiments, the receiving frame may form a part of the longitudinal shaft of the instrument.

For illustrative purposes, the terms longitudinal, lateral and vertical will be used herein to describe relative orientations of the components of the distal head assembly. However, this shall not be construed as limiting the scope. For illustrative purposes, the longitudinal direction is generally aligned with the main extension direction of the elongated shaft to which the distal head assembly may be attached. For illustrative purposes, the lateral direction is basically perpendicular to the longitudinal direction and may be defined by a movement axis (pivoting axing) of the the movable jaw arrangement. In the alternative, the lateral axis may indicate the direction in which the bearing portions of the first lateral arm and the second lateral arm are offset from one another. Further, the vertical direction may be referred to as being perpendicular to the longitudinal direction and to the lateral direction. It goes without saying, that the above-indicated directions are primarily associated with the bipolar instrument, particularly the distal head assembly that can be attached thereto. As a consequence, these directions do not necessarily represent absolute environmental longitudinal, lateral and vertical directions.

A further benefit of this arrangement is that the required number of parts for the distal head assembly can be greatly reduced. Hence, manufacturing and assembling efforts may be reduced accordingly. Further, since fewer parts are present, accuracy and precision of the distal head assembly may be further enhanced. Further, the risk of mounting failures resulting in incorrect fitting and mounting may be reduced.

In one exemplary embodiment of the assembly, the jaw arrangement comprises a first jaw and a second jaw, wherein the first jaw is mounted to the first lateral arm, wherein the second jaw is mounted to the second lateral arm, wherein a mounting portion of the transmitting piece is interposed between the first jaw and the second jaw, and wherein the jaw arrangement is pivotably supported at the receiving frame in a positive-locking fashion. In accordance with this embodiment, a lateral order of parts may be as follows: first lateral arm, first jaw, transmitting piece, second jaw, and second lateral arm.

There are two main types of movable jaw arrangements for bipolar instruments. In a first type, both the first jaw and the second jaw are movable with respect to the receiving frame and, as a consequence, with respect to one another. In another type, only one of the first jaw and the second jaw is movable with respect to the other (fixed) jaw. In both arrangements, the first jaw and the second jaw may be jointly positioned in a first, closed state and a second, opened state.

In a further exemplary embodiment of the assembly, at least one of the first lateral arm and the second lateral arm is deflectable to allow the jaw arrangement to engage the receiving portion of the receiving frame, wherein the retaining bracket, in the mounted state, retains the first lateral arm and the second lateral arm in a basically non-deflected state. To accomplish the assembling procedure, at least one of the first lateral arm and the second lateral arm is basically temporarily outwardly deflected so as to provide sufficient assembly space. Once the transmitting piece and the jaw arrangement are positioned at the receiving portion, the at least one outwardly deflected lateral arm flexes back into the original unbiased position. Hence, co-operating mounting elements may engage one another so as to lock the components in a positive-locking fashion.

The retaining bracket prevents the lateral arms of the receiving frame from being re-deflected and therefore prevents the jaw arrangement and the transmitting piece from being disengaged from the receiving frame. The retaining bracket locks, covers or guards a distal end of the receiving frame and therefore also forms part of a surrounding mounting base or frame base for a pivoting mechanism that enables the movement of the jaw arrangement. Hence, the retaining bracket further ensures positional accuracy and operational accuracy. In the non-deflected state, basically no bending stresses are present at the first lateral arm and the second lateral arm. In other words, the non-deflected state may be referred to as relieved state. By way of example, in the deflected state, the arms of the receiving frame are bent apart from one another.

According to the invention, the retaining bracket is arranged to be attached to a frontal end of the receiving frame. Hence, the retaining bracket is referred to as frontal locking bracket.

In yet another embodiment of the assembly, the retaining bracket comprises a base, a first retaining arm and a second retaining arm extending therefrom in a basically longitudinal direction, wherein the first retaining arm and the second retaining arm are laterally spaced from one another, wherein the receiving frame comprises a first base groove arranged at the first lateral arm and a second base groove arranged at the second lateral arm, wherein the first base groove and the first retaining arm engage one another in the mounted state, and wherein the second base groove and the second retaining arm engage one another in the mounted state. Hence, when viewed in a plane perpendicular to the vertical direction, the retaining bracket basically comprises a U-shaped profile. Consequently, the retaining bracket may also be referred to, at least in some embodiments, as chin guard for the front end of the receiving frame.

In a refinement of the above embodiment, the first base groove and the first retaining arm define a longitudinally extending dovetail joint, wherein the second base groove and the second retaining arm define a longitudinally extending dovetail joint. This has the further advantage that the retaining bracket is not only preventing the lateral arms of the receiving frame from being outwardly deflected, but also from being inwardly flexed or deflected. This further improves rigidity and stiffness of the distal head assembly. The assembly's shape retention is further improved.

As used herein, a dovetail joint comprises mating elements including a base groove providing a non-rectangular or inwardly tapered cross-section (when viewed in a plane that is perpendicular to the longitudinal direction). The cross-section of the slot profile base at the grooves may be therefore trapezoid, triangular and similarly shaped. Generally, the base grooves may comprise inclined side walls, wherein an acute angle is provided between a side wall and a ground wall of the base groove. As a consequence, the position of a counterpart of the base groove, such as the retaining arms of the retaining bracket, is defined in the lateral direction in a bidirectional fashion. In other words, the retaining arms are prevented from being lifted or detached from the base groove in any lateral and vertical direction.

In yet another exemplary embodiment of the assembly, at least one locking pin is provided which is arranged to secure the mounted state of the mounting bracket. The locking pin may at least partially extend through one of the retaining arms of the mounting bracket and one of the lateral arms of the receiving frame. Preferably, the at least one locking pin further engages at least one of the jaws of the distal head assembly. The locking pin may be arranged to be laterally inserted in respective locking recesses so as to prevent a removal of the (mounted) retaining bracket in the longitudinal direction. The locking pin may therefore act as a loss prevention member.

In yet another embodiment of the assembly, the jaw arrangement comprises a first bearing element and a second bearing element, the first bearing element engaging the first lateral arm and the second bearing element engaging the second lateral arm, wherein the first bearing element and the second bearing element define a pivot axis. Preferably, the first bearing element is arranged at the first jaw to define a pivot axis thereof, wherein the second bearing element is arranged at the second jaw to define a pivot axis thereof. In some embodiments, when two movable jaws are implemented, the respective pivot axes are parallel to one another. In some embodiments, the first bearing element and the second bearing element are arranged in a fashion coincident with one another and therefore define a common pivot axis of the jaw arrangement.

As already indicated above, there may be further embodiments wherein the jaw arrangement comprises a movable jaw and a stationary jaw, wherein the movable jaw is pivotable with respect to the stationary jaw. However, in accordance with further embodiments as discussed herein, the jaw arrangement comprises a first movable jaw that pivots about a first pivot axis and a second movable jaw that pivots about a second pivot axis wherein the first movable jaw and the second movable jaw are preferably arranged to pivot about a common overall pivot axis.

In a further refinement of the above embodiment, the first bearing element is integrally formed at the first jaw and engages a mounting recess at the first lateral arm, wherein the second bearing element is integrally formed at the second jaw and engages a mounting recess at the second lateral arm. Hence, the number of components for the distal head assembly can be even further reduced. The bearing elements may be so-to-say fixed to the first jaw and the second jaw since the receiving frame is at least partially deflectable so as to enable the mounting action. It is not necessary to attach separate bearing elements such as bearing pins to the first jaw and the second jaw, as may be the case with conventional distal head assemblies that do not implement deflectable receiving portions.

In still another embodiment of the assembly, the transmitting piece comprises a first elongated slot arranged at a first lateral side thereof and a second elongated slot arranged at an opposite second lateral side thereof, wherein the first jaw comprises a first mounting protrusion engaging the first elongated slot, wherein the second jaw comprises a second mounting protrusion engaging the second elongated slot, wherein the first jaw and the second jaw are arranged to be inserted into the receiving portion, and wherein a main elongation direction of the first elongated slot and the second elongated slot is aligned with the longitudinal direction.

The mounting protrusions are preferably aligned with the bearing elements. In one embodiment, the respective bearing element and the mounting protrusion of the first jaw and the second jaw are arranged at respective opposite sides thereof. In accordance with this embodiment, the mounting protrusion and the bearing element are arranged in a concentric fashion. Also the mounting protrusions of the first jaw and the second jaw are preferably integrally formed with the jaws.

In operation, the transmitting piece transmits a longitudinal actuation movement to the jaws of the jaw arrangement. Typically, the transmitting piece is arranged in a reciprocatingly movable fashion at the receiving frame. Therefore, the slots arranged at the transmitting piece for receiving the mounting protrusions of the jaws are preferably elongated. Hence, in operation, relative movement of the jaw arrangement and the transmitting piece involves a pivoting movement of the jaw arrangement with respect to the transmitting piece and a reciprocating transversal movement of the transmitting piece with respect to the jaw arrangement.

In a further embodiment of the assembly, the first jaw and the second jaw are arranged at the receiving frame and the transmitting piece in a fashion pivotable with respect to one another between a first pivot state and a second pivot state about a laterally extending pivot axis defined by the receiving frame, wherein operating ends of the first jaw and the second jaw approach one another in the first pivot state and are spaced away from one another in the second pivot state, and wherein the first jaw and the second jaw jointly encompass the retaining bracket in the first pivot state. The first pivot state may involve a contact between the operating ends of the first jaw and the second jaw.

Hence, the first jaw and the second jaw may be operated in a scissor-like fashion to effect a clamping, pressing or cutting action. At the operating ends of the first jaw and the second jaw, contact surfaces may be arranged which contact the to-be-treated tissue portion or vessel. Needless to say, the first pivot state and the second pivot state do not necessarily define a respective distinct relative position but rather a first range and a second range of the relative pivoting movement.

In yet another embodiment, the distal head assembly further comprises a mechanical linkage arranged to transfer a longitudinal movement of the transmitting piece into a swiveling movement of the movable jaw arrangement, preferably a pivoting movement of the first jaw and the second jaw in opposite direction, wherein the mechanical linkage comprises two opposite slide joints including a first slide joint that couples the transmitting piece and the first jaw, and a second slide joint that couples the transmitting piece and the second jaw, and wherein the first slide joint and the second slide joint are oriented in an opposite inclination with respect to a longitudinal axis of the transmitting piece.

The inclined slide joints may be referred to as inclined lift-shaped arrangements. The longitudinal axis of the transmitting piece is basically parallel to the movement direction of the transmitting piece. Since the first slide joint and the second slide joint are inclined in opposite fashion, a longitudinal driving movement of the transmitting piece may effect an opposite swiveling movement of the first jaw and the second jaw.

Generally, the first slide joint and the second slide joint may be arranged as non-parallel slides. In some embodiments, the first slide and the second slide may be arranged as a cross slide. However, an inclination angle between the first slide and the second slide is, in accordance with some embodiments, an acute angle. Since also the transmitting piece basically forms the carriage of a further (longitudinal) slide joint, in total three slide joints may be present. Preferably, three non-parallel slide joints are present, wherein the slide joint of the transmitting piece (and the receiving frame) is aligned with the longitudinal axis thereof, and wherein the first slide joint is arranged at a first angle of inclination with respect to the longitudinal axis, wherein the second slide joint is arranged at an angle of inclination with respect to the longitudinal axis of the transmitting piece, and wherein the inclination angels of the first slide joint and the second slide joint are of the same size but arranged in opposite fashion.

In a refinement of the above embodiment, the first slide joint comprises a first slide piece movably arranged at a first slide base of the transmitting piece, wherein the second slide joint comprises a second slide piece movably arranged at the second slide base of the transmitting piece, wherein the first slide piece engages a coupling portion of the first jaw for driving the first jaw and wherein the second slide piece engages a coupling portion of the second jaw for driving the second jaw.

Therefore, in accordance with a further embodiment, the mechanical linkage of the distal head assembly consists of the transmitting piece which is arranged at the receiving frame, the first jaw and the second jaw which are also arranged at the receiving piece, and the first slide piece and the second slide piece, wherein the first slide piece is interposed between the transmitting piece and the first jaw, and wherein the second slide piece is interposed between the transmitting piece and the second jaw. Also the mechanical linkage only requires a limited number of components which further facilitates manufacturing and assembling the distal head assembly.

In accordance with a further embodiment, at least one drain groove is provided between the transmitting piece and the receiving frame. Preferably, a first lateral drain groove and a second lateral drain groove is provided. The at least one drain groove may be formed at the transmitting piece, preferably in the vicinity of the slide bases of the slide joints. The at least one drain groove may extend in the vertical direction.

In still another refinement of the mechanical linkage, the first slide base is a slide groove arrangement at the transmitting piece, wherein also the second slide base is a slide groove arranged at the transmitting piece at an opposite side thereof, wherein the first slide piece comprises a protruding engagement stud engaging a mating coupling recess at the coupling portion of the first jaw, and wherein the second slide piece comprises a protruding engagement stud engaging a mating coupling recess at the coupling portion of the second jaw. In this way, a conversion or translation of a single reciprocating longitudinal movement into an output movement that involves a first pivoting movement of the first jaw and a second pivoting movement of the second jaw can be achieved with relatively little installation space and efforts.

In a further embodiment of the mechanical linkage, at least one of the mating coupling recesses of the first jaw and the second jaw is arranged as a slot comprising an open end. This may further facilitate manufacturing and assembling the mechanical linkage.

In another embodiment of the distal head assembly, the first jaw and the second jaw, particularly the operating ends thereof, are isolated from one another in a non-contact state of the operating ends. Hence, there is no direct lead connection or (electrical) power connection between the operating ends of the first jaw and the second jaw. In practice, when a tissue portion or blood vessel is clamped between the first jaw and the second jaw, the human or animal tissue/vessel acts as an conductor that (electrically) connects the first jaw and the second jaw.

In particular, the receiving frame is at least substantially formed from isolating material, preferably from isolating elastic material, particularly from plastic material. In one embodiment, at least the first lateral arm and the second lateral arm of the receiving frame are obtained from injection-moldable plastic material. Hence, the first lateral arm and the second lateral arm are, at least to some extent, deflectable so as to provide sufficient assembly space for the to-be-mounted transmitting piece and the movable jaw arrangement. Further, also the retaining bracket is made from isolating material, particularly from plastic material. As used herein, isolating material involves at least one of the group consisting of plastic material, thermosetting material, thermoplastic material, ceramic material, and elastomer material.

In another aspect of the present disclosure, a bipolar instrument, particularly a coagulating instrument selected from the group consisting of bipolar forceps, bipolar scissors, a bipolar clamp and bipolar pincers, is presented, the instrument comprising a distal head assembly in accordance with at least one embodiment as disclosed herein. Preferably, the instrument comprises an elongated shaft at a distal end of which the head assembly is arranged. Further, the elongated shaft may house at least one transmission element that applies an actuating movement or force to the transmitting piece. Further, the elongated shaft may house lead connectors for contacting the first jaw and the second jaw which act as electrodes. At a proximal end of the elongated shaft which is opposite to the distal end, a handling unit may be provided. Preferably, the instrument is arranged as an endoscopic or laparoscopic instrument. Generally, the instrument may be suitable for open surgery treatment, but also for non-invasive surgery treatment.

In yet another aspect of the present disclosure, a method of assembling a distal head assembly for a bipolar instrument is presented, the method comprising the steps of:
- providing a receiving frame comprising a first lateral arm and a second lateral arm that define therebetween a receiving portion,
- providing a transmitting piece and a movable jaw arrangement comprising a first jaw and a second jaw,
- arranging the first jaw and the second jaw at the transmitting piece,
- inserting the sub-assembled transmitting piece and the jaw arrangement in the receiving portion of the receiving frame in a snap-on mounting fashion, comprising temporarily deflecting at least one of the first lateral arm and the second lateral arm, wherein the first jaw engages the transmitting piece and the first lateral arm, and wherein the second jaw engages the transmitting piece and the second lateral arm, and
- securing the mounted state of the jaw arrangement by attaching a retaining bracket to the receiving frame.

In one embodiment of the assembly method, the retaining bracket is attached to the receiving frame, particularly to the first lateral arm and the second lateral arm thereof, in a basically non-releasable fashion. This may involve a molding, force-fit or positive-fit action.

In a further embodiment of the assembly method, the step of arranging the first jaw and the second jaw at the transmitting piece further comprises:
- mounting at least one slide piece in a slide groove at the transmitting piece,
- arranging at least one of the first jaw and the second jaw in a respective mounting orientation, and
- approaching the transmitting piece with the jaw, including engaging the slide piece with a coupling recess of the jaw and an elongated slot arranged at a lateral side of the transmitting piece with a mounting protrusion of the jaw.

In a further embodiment of the above assembly method, the step of securing the mounted state of the jaw arrangement by attaching a retaining bracket to the receiving frame further comprises:
- attaching the retaining bracket to a frontal end of the receiving frame, including engaging at least one base groove of the receiving frame with at least one retaining arm of the the retaining bracket, wherein the at least on base groove and the at least one retaining arm preferably form a longitudinally extending dovetail joint.

It goes without saying that features of the present disclosure that have been mentioned hereinbefore and will be described hereafter can be used not only in the respectively specified combination, but also in other combinations or in isolation without departing from the scope of the present disclosure.

Further features and exemplary embodiments of the present disclosure are disclosed in the following description of exemplary embodiments, with reference to the drawings, wherein:
- Fig. 1: is a perspective frontal view of an exemplary embodiment of a distal head assembly of a bipolar instrument, the head assembly shown in a first state;
- Fig. 2: is a further view of the arrangement of Fig. 1, wherein the head assembly is shown in a second state;
- Fig. 3: is an exploded perspective frontal view of the arrangement of Fig. 2;
- Fig. 4: is a further exploded view of the arrangement of Fig. 2 in a viewing orientation that is different from that of the view of Fig. 3;
- Fig. 5: is a lateral side view of the arrangement of Fig. 1;
- Fig. 6: is a perspective cross-sectional view of the arrangement of Fig. 1, wherein a view orientation corresponds to the view of Fig. 1, and wherein the cross-sectional plane is indicated in Fig. 5 by a line VI-VI;
- Fig. 7: is a perspective cross-sectional rear view of the arrangement of Fig. 2, wherein a cross-sectional plane is indicated by line VII-VII in Fig. 6;
- Fig. 8: is a front view of the arrangement of Fig. 7;
- Fig. 9: is perspective front view of a subassembly of an exemplary embodiment of a distal head assembly;
- Fig. 10: is perspective front view of a retaining bracket for the subassembly of Fig 9;
- Fig. 11: is a further perspective front view of the arrangement of Fig. 9, wherein the distal head assembly is in a further assembled state;
- Fig. 12: is a simplified broken view of another embodiment of a bipolar instrument; and
- Fig. 13: is a schematic flow chart illustrating an exemplary embodiment of a method of assembling a distal head assembly for a bipolar instrument.

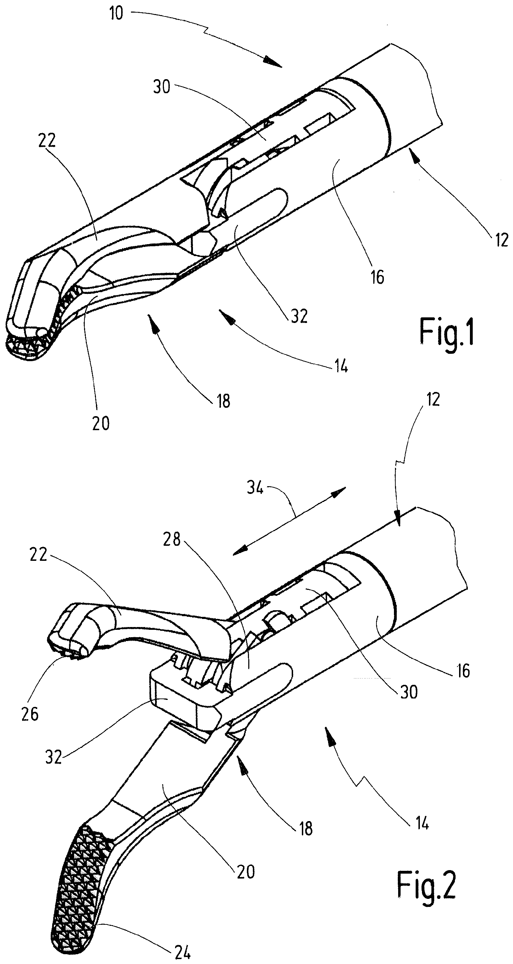

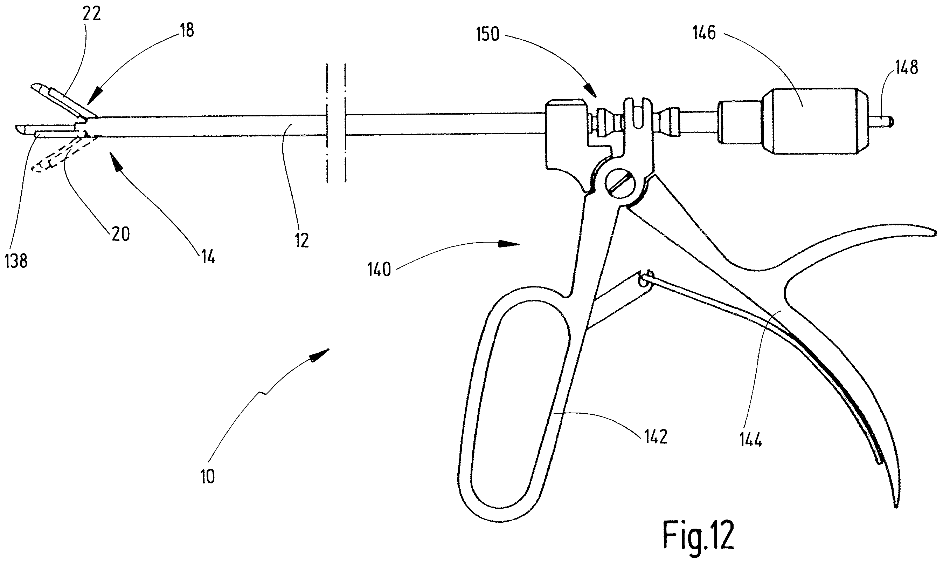

A distal head assembly is shown in a perspective frontal view in Fig. 1 and designated by reference numeral 14. The head assembly 14 is arranged to be attached to a shaft 12 of a bipolar instrument 10. It is context, reference is made to Fig. 12 showing a partial view of an exemplary embodiment of a bipolar instrument 10 which is arranged as bipolar forceps.

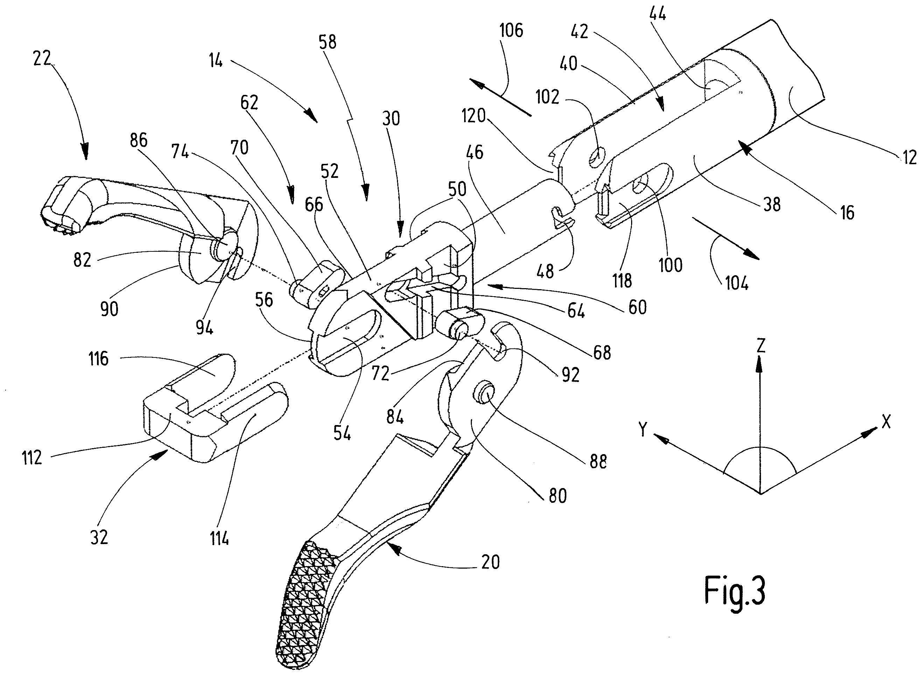

With reference to Figs. 1, 2 and 3, an exemplary embodiment of a head assembly 14 in accordance with at least some aspects of the present disclosure will be further described and elucidated. As shown in at least some Figures herein, for illustrative purposes, a coordinate system (Cartesian coordinate system) X-Y-Z is provided, refer to Fig. 3, for instance. The coordinate system X-Y-Z is used in the following for describing orientations and locations of components of the head assembly 14. It goes without saying that the skilled person is capable of adapting or, if necessary, transforming or converting the coordinate system X-Y-Z when being confronted with new embodiments, illustrations and/or orientations as the coordinate system X-Y-Z is merely an illustrative means for describing elements of the presented exemplary embodiment of the head assembly 14 and their interrelation.

The head assembly 14 is arranged at a distal end of the shaft 12 of the instrument 10. The shaft 12 comprises a main elongation extension which will be hereinafter associated with the X-axis (longitudinal direction). Further, for illustrative purposes, the Y-axis will be associated with a pivoting axis of the head assembly 14. The X-axis may also be referred to as lateral axis or direction. Further, the Z-axis will be associated with the vertical direction herein. The Z-direction is basically perpendicular to a plane that is jointly defined by the X-axis and the Y-axis. In the following, the distal end of the shaft 12 to which the head assembly 14 is attached, will be also referred to as frontal end.

The head assembly 14 comprises a receiving frame 16 which is coupled to the shaft 12. When viewed in a plane that is perpendicular to the Z-direction, the receiving frame 16 has a basically U-shaped profile, refer also to the exploded view of Fig. 3.

Further, the head assembly 14 comprises a jaw arrangement 18 which is mounted to the receiving frame 16 in a movable fashion, particularly a pivotable fashion. The pivoting movement of the jaw arrangement 18 is indicated in Fig. 1 and Fig. 2, wherein Fig. 1 illustrates a first state, and wherein Fig. 2 illustrates a second state. The jaw arrangement 18 comprises a first jaw 20 and a second jaw 22. As already discussed hereinbefore, in some exemplary embodiments, the jaw arrangement 18 comprises a stationary jaw and a movable jaw. However, as with the embodiment illustrated in Figs. 1, 2 and 3, both the first jaw 20 and the second jaw 22 are movable. Consequently, the first state of Fig. 1 may be regarded as a closed state or approaching state of the first jaw 20 and the second jaw 22. By contrast, the second state as indicated in Fig. 2 may be regarded as an opened state or a spaced away state of the first jaw 20 and a second jaw 22.

At the first jaw 20, a first operating end 24 is provided. At the second jaw 22, a second operating end 26 is provided. When in operation, the first operating end 24 and the second operating end 26 may clamp a tissue portion or a blood vessel of a to-be-treated subject therebetween to transfer electrical energy therethrough. In this way, a coagulating treatment may be performed. Hence, the first operating end 24 and the second operating end 26 may be referred to as operating electrodes 24, 26. At a frontal end 28 of the receiving frame 16 that is facing away from the shaft 12, the jaw arrangement 18 may be received and mounted in a pivotable fashion.

The head assembly 14 further comprises a transmitting piece 30, refer particularly to Fig. 3. The transmitting piece 30 is arranged to transmit an actuating movement to the first jaw 20 and the second jaw 22 to effect the pivoting movement thereof. As indicated by a double-arrow 34 in Fig. 2, the transmitting piece 30 is arranged at the receiving frame 16 in a reciprocatingly movable fashion. Further, a retaining bracket 32 is attached to the receiving frame 16 at the frontal end 28 thereof. The retaining bracket 32 so-to-say locks the transmitting piece 30 and the jaw arrangement 18 in their mounting position at the receiving frame 16.

With particular reference to Fig. 3, the head assembly 14 will be described and elucidated in more detail. The receiving frame 16 comprises a first lateral arm 38 and a second lateral arm 40. The first lateral arm 38 and the second lateral arm 40 are spaced from one another in the lateral direction Y. Between the first lateral arm 38 and the second lateral arm 40, a receiving portion 42 is provided in which the transmitting piece 30 and the jaw arrangement 18 including the first jaw 20 and the second jaw 22 are arranged in the mounted state of the head assembly 14.

Further, a rear transmitting slot 44 is provided at the receiving frame 16. In the mounted state, a support shaft 46 of the transmitting piece 30 is inserted into and extends through the transmitting slot 44. At a rear end of the support shaft 46, a fastening portion 48 is provided to which an actuation force can be applied to effect the pivoting movement of the jaw arrangement 18. By way of example, the fastening portion 48 may form part of a bayonet joint. Since the transmitting piece 30 is basically arranged to be reciprocatingly moved in the longitudinal direction X, the fastening portion 48 is arranged to transfer a push-and-pull movement. Further, the support shaft 46 is at least partially provided with a recess in which lead connectors may be arranged to contact the first jaw 20 and the second jaw 22.

Between the receiving frame 16 and the transmitting piece 30, drain slots or drain grooves 50 are provided. In one exemplary embodiment, the drain grooves 50 are arranged at opposite lateral sides of the transmitting piece 30. The drain grooves 50 provide for a drain clearance between the transmitting piece 30 and the lateral arms 38, 40 of the receiving frame 16. When the distal head assembly 14 is in operation, it may not be unlikely that a fluid (a liquid) contacts and adheres to components thereof. This may cause (temporary) short circuits and may thus mitigate the performance of the distal head assembly 14. The drain grooves 50 may basically extend in the vertical direction (Z direction).

Adjacent to the support shaft 46, the transmitting piece 30 comprises a mounting portion 52. When the transmitting piece 30 and the first jaw 20 and the second jaw 22 are in the mounted configuration, the first jaw 20 is arranged between the first lateral arm 38 and the mounting portion 52. Further, the second jaw 22 is arranged between the second lateral arm 40 and the mounting portion 52. Consequently, the mounting portion 52 is arranged between the first jaw 20 and the second jaw 22.

At a frontal end of the mounting portion 52, a first elongated slot 54 and a second elongated slot 56 is provided, the second elongated slot 56 being arranged opposite to the first elongated slot 54. The elongated slots 54, 56 are spaced from one another in the lateral direction Y. Preferably, the transmitting piece 30 is a basically integrally shaped component of the head assembly 14.

The head assembly 14 further comprises a mechanical linkage 58 which is arranged to transmit a longitudinal actuating movement of the transmitting piece 30 into the desired pivoting movement of the jaw arrangement 18. By way of example, the mechanical linkage 58 comprises a first slide joint 60 and a second slide joint 62 (only partially visible in Fig. 3) arranged at respective opposite lateral sides of the mounting portion 52 of the transmitting piece 30. Further, a first slide base 64 and a second slide base 66 are arranged at the mounting portion 52 at respective opposite sides thereof. The slide bases 64, 66 are arranged as slide grooves, for instance. The slide bases 64, 66 are arranged at an angle of inclination with respect to the longitudinal direction X. The slide basis 64, 66 are inclined with respect to the longitudinal direction X in an opposite fashion. The first slide base 64 is associated with the first slide joint 60. The second slight base 66 (hidden in Fig. 3) is associated with the second slide joint 62.

The first slide joint 60 further comprises a first slide piece 68. The second slide joint 62 further comprises a second slide piece 70. The slide pieces 68, 70 may be referred to as carriages. The first slide piece 68 is arranged to be received at and moved along the first slide base 64. The second slide piece 70 is arranged to be received at and moved along the second slide base 66. The slide pieces 68, 70 may also be referred to as slide stones. At the first slide piece 68, a first engagement stud 72 is provided. At the second slide piece 70, a second engagement stud 74 is provided. When the transmitting piece 30 is moved in the longitudinal direction X, the slide pieces 68, 70 are basically moved in the vertical direction Z, due to the inclination of the slide bases 64, 66. Since the angle of inclination of the first slide base 64 is opposite to the angle of inclination of the second slide base 66, the first slide piece 68 and the second slide piece 70 are moved in an opposite fashion along the Z-direction.

The slide bases 64, 66 may cross or extend through the drain grooves 50. The slide bases 64, 66 are movably arranged and may therefore wipe or distribute a fluid or liquid that may enter a gap between the transmitting piece 30 and the lateral arms 38, 40 which may eventually cause short circuits between the first jaw 20 and the second jaw 22. Further, as also the transmitting piece 30 may be movably (slidably) arranged with respect to the receiving frame 16, also the relative longitudinal movement between the transmitting piece 30 and the receiving frame 16 may somewhat wipe or distribute any liquid therebetween. Hence, the drain grooves 50, in some respect, interrupt a gap between the transmitting piece 30 and the lateral arms 38, 40 and may therefore reduce the risk of short circuits.

At the jaw arrangement 18, a first coupling portion 80 is provided at the first jaw 20 and a second coupling portion 82 is provided at the second jaw 22. At the first coupling portion 80, a first mounting protrusion 84, a first bearing element 88 and a first coupling recess 92 is provided. At the second coupling portion 82 of the second jaw 22, a second mounting protrusion 86, a second bearing element 90 (hidden in Fig. 3) and a second coupling recess 94 is provided. Preferably, the first mounting protrusion 84 and the first bearing element 88 are arranged in concentric fashion. Further, the second mounting protrusion 86 and the second bearing element 90 are preferably arranged in concentric fashion.

The first mounting protrusion 84 and the first bearing element 88 define a first pivot axis of the first jaw 20. The second mounting protrusion 86 and the second bearing element 90 define a second pivot axis of the second jaw 22. In the embodiment of Figs. 1 to 3, a common pivot axis for the first jaw 20 and the second jaw 22 is provided which extends parallel to the Y-direction. Hence, the first jaw 20 and the second jaw 22 pivot about this axis when the transmitting piece 30 is moved in the actuation direction 34. The first engagement stud 72 of the first slide piece 68 engages the first coupling recess 92. The second engagement stud 74 of the second slide piece 70 engages the second coupling recess 94. Since the coupling recesses 92, 94 of the jaws 20, 22 are radially spaced away from the respective pivot axis, a force application lever is provided to induce the pivoting movement.

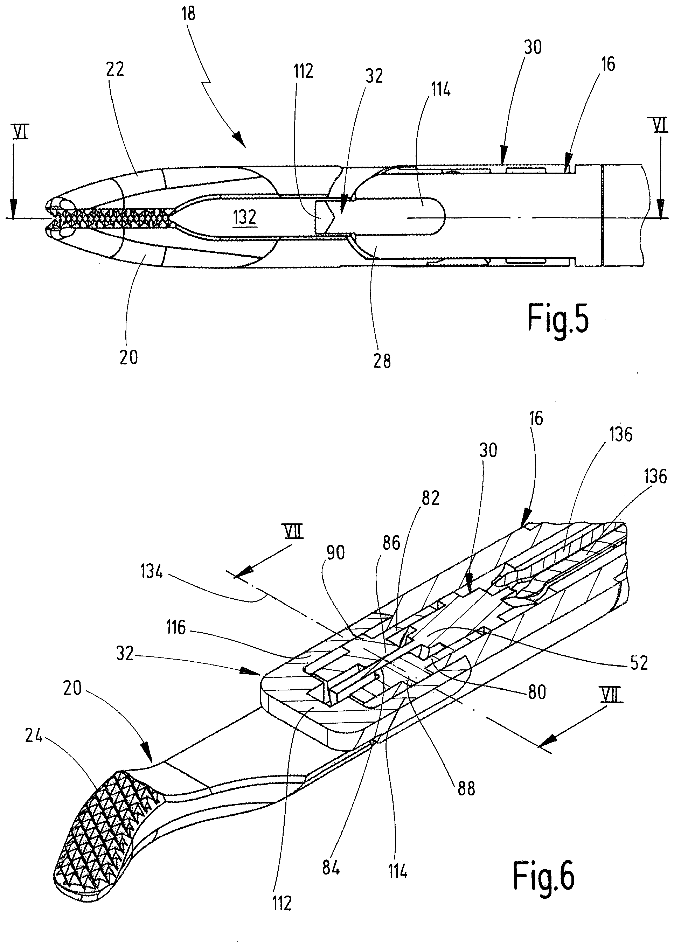

At the first lateral arm 38 of the receiving frame 16, a first mounting recess 100 is provided. At the second lateral arm 40, a second mounting recess 102 is provided. In the mounted state, the first bearing element 88 of the first jaw 20 engages the first mounting recess 100. Similarly, the second bearing element 90 (refer to Fig. 6) engages the second mounting recess 102 in the mounted state. Hence, the mounting recesses 100, 102 which are aligned to one another in the embodiment of Figs. 1 to 3 define a common overall pivot axis for the first jaw 20 and the second jaw 22.

The first jaw 20 and the second jaw 22 are pivotably supported at the receiving frame 16, wherein the pivot axis is defined by the bearing elements 88, 90 and their counterpart mounting recesses 100, 102. The transmitting piece 30 is arranged for a reciprocating longitudinal movement. Therefore, the slots 54, 56 at the transmitting piece 30 are arranged as elongated slots. Hence, when the transmitting piece 30 is moved between an extracted and an retracted state, and when the jaw arrangement 18 is correspondingly pivoted between the first state and the second state, also the elongated slots 54, 56 are linearly moved in the longitudinal direction X, whereas their counterparts, the mounting protrusions 84, 86 remain in their longitudinal position but are pivoted about the pivot axis which is parallel to the Y-axis.

Assembling the head assembly 14 may involve assembling a sub-assembly involving the transmitting piece 30, the first slide piece 68, the second slide piece 70, the first jaw 20 and the second jaw 22, and inserting the sub-assembled components into the receiving portion 42 of the receiving frame 16. To this end, at least one of the first lateral arm 38 and the second lateral arm 40 is deflectable. In Fig. 3, a mounting deflection direction of the first lateral arm 38 is indicated by an arrow 104. Further, a mounting deflection direction of the second lateral arm 40 is indicated by an arrow 106. When at least one of the first lateral arm 38 and the second lateral arm 40 is sufficiently outwardly deflected, the sub-assembled components may pass a mounting gap that is provided in this way and enter the receiving portion 52. The first bearing element 88 may engage the first mounting recess 100, and the second bearing element 90 may engage the second mounting recess 102. Consequently, any deflected first lateral arm 38 or second lateral arm 40 may flex back into the original orientation. Consequently, a positive-fit locking engagement maintains the mounted operable state the head assembly 14.

So as to ensure that the locking engagement between the first lateral arm 38 and the second lateral arm 40 and the respective first and second jaw 20, 22 is maintained, which involves that also the engagement between the jaws 20, 22 and the transmitting piece 30 is maintained, the retaining bracket 32 is attached to the receiving frame 16 at the frontal end 28 thereof. In the mounted state, the retaining bracket 32 prevents the first lateral arm 38 and the second lateral arm 40 from being outwardly deflected. Consequently, the transmitting piece 30, the first jaw 20 and the second jaw 22 and the slide pieces 68, 70 that are interposed therebetween, cannot be brought out of engagement with one another.

In the exemplary embodiment of Figs. 1 to 3, the retaining bracket 32 comprises a base 112, a first retaining arm 114 and a second retaining arm 116. Consequently, when viewed in a plane that is perpendicular to the vertical direction Z, the retaining bracket 32 comprises a basically U-shaped arrangement. The retaining bracket 32 may be moved and brought into engagement with the receiving frame 16 in the longitudinal direction X. To this end, a first base groove 118 is arranged at the first lateral arm 38, and a second base groove 120 is arranged at the second lateral arm 40 at the frontal ends thereof. The first base groove 118 and the second base groove 120 are outwardly facing and arranged in opposite fashion.

As with the embodiment of Fig. 3, the first mounting recess 100 extends through the first lateral arm 38 in the lateral direction Y. Further, the first mounting recess 100 runs into the first base groove 118. Similarly, the second mounting recess 102 extends through the second lateral arm 40 in the lateral direction Y and runs into the second base groove 120, refer also to Fig. 6. Hence, the retaining arms 114, 116 cover the bearing elements 88, 90 of the jaws 20, 22 which are arranged in the respective mounting recesses 100, 102 in the mounted state.

Preferably, the retaining arms 114, 116 and the respective counterpart base grooves 118, 120 are arranged in a dovetail-like fashion. As a consequence, the retaining bracket 32 does not only prevent an outward deflection of the first lateral arm 38 and the second lateral arm 40, but also an inward deflection thereof. More generally, the retaining bracket 32 prevents relative lateral flexing between the first arm 38 and the second arm 40. This may have the effect that for instance a desired mounting clearance can be maintained which is crucial for the function of the mechanical linkage 58. Hence, ease of movement of the jaw arrangement 18 may be ensured.

Preferably, the retaining bracket 32 is locked at or secured to the receiving frame 16 in a loss-proof fashion. To this end, the retaining bracket 32 may be crimped, pressed-in, molded, bonded or similarly fixedly attached to the receiving frame 16.

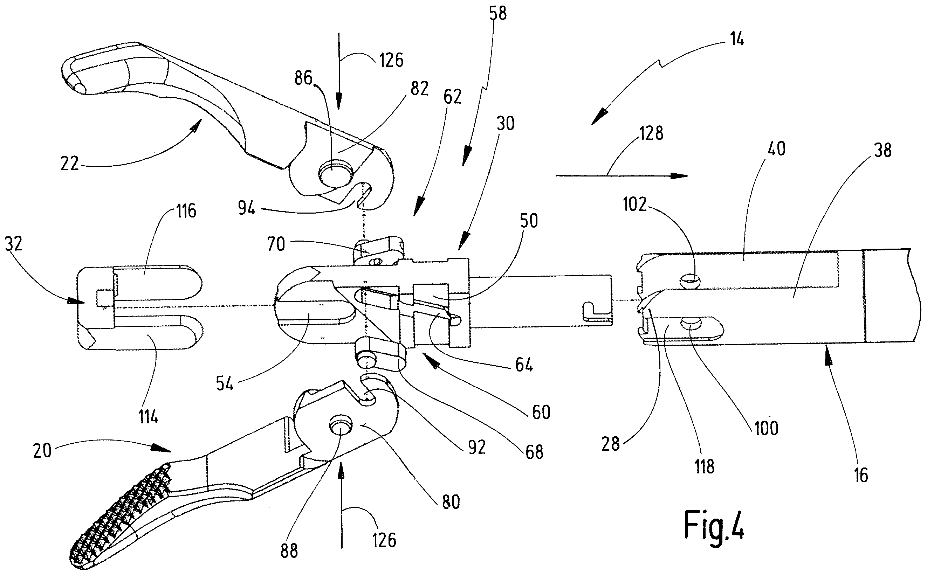

With additional reference to Fig. 4, an exemplary mounting or assembly procedure for the distal head assembly 14 is elucidated. As indicated above, initially, a sub-assembly is formed which involves the transmitting piece 30, the jaw arrangement 18, and the first slide piece 68 and the second slide piece 70 that are interposed between the transmitting piece 30 and the respective jaws 20, 22 of the jaw arrangement 18. For instance, the coupling portions 80, 82 may be brought into an assembly orientation wherein, when viewed in a plane that is perpendicular to the lateral direction Y, the mounting protrusions 84, 86 and the coupling recesses 92, 94 are in registry or congruent with their counterparts, the elongated slots 54, 56 at the mounting portion 52 and the engagement studs 72, 74 of the slide pieces 68, 70 that are arranged at the slide basis 64, 66, respectively.

Once the assembly orientation is assumed, the mounting or engagement may be accomplished. A respective mounting direction is indicated in Fig. 4 by the arrows 126. Accordingly, the mounting direction is basically parallel to the lateral direction Y. Once the sub-assembly containing the retaining bracket 32, the slide piece 68, the slide piece 70 and the jaw arrangement 18 is accomplished, the sub-assembly may be inserted in the mounting portion 52 between the first lateral arm 38 and the second lateral arm 40, provided that at least one of the arms 38, 40 is outwardly deflected as indicated by the arrows 104, 106 in Fig. 3. The respective mounting direction is indicated in Fig. 4 by an arrow designated by reference numeral 128. Assembling the distal head assembly 14 involves a snap-on or clip-on mounting operation. Eventually, the retaining bracket 32 may be attached to the frontal end 28 of the receiving frame 16 so as to secure and accomplish the assembling operation.

Further reference is made to Fig. 5, Fig. 6, Fig. 7 and Fig. 8 which illustrate the mounted state of the distal head assembly 14 in more detail. Fig. 5 is a lateral view of the head assembly 14 in the first state, where the first jaw 20 and the second jaw 22 are shown in an approaching state basically contacting one another. It goes without saying that in operation typically a blood vessel or a piece of tissue of a to-be-treated subject is interposed between the first jaw 20 and the second jaw 22. As can be further seen from Fig. 5, the first jaw 20 and the second jaw 22 span over the retaining bracket 32, particularly over the base 112 thereof. In other words, the jaw arrangement 18 bridges the retaining bracket 32 in the lateral view. Adjacent to the base 112 of the retaining bracket 32, a gap 132 is defined by the jaw arrangement 18 in the first, approaching state. The retaining bracket 32 is arranged between the pivot axis and the operating ends 24, 26 of the jaws 20, 22.

Fig. 6 shows a perspective cross-sectional view oft he head assembly 14, wherein the sectional plane is indicated in Fig. 5 by VI-VI. As can be seen from Fig. 6, the bearing elements 88, 90 of the jaws 20, 22 of the jaw arrangement define a common pivot axis 134 which is basically extending in the lateral direction Y. The first jaw 20 and the second jaw 22 swivel or pivot about the pivot axis 134 in a defined fashion when operated via the transmitting piece 30 and the mechanical linkage 58 that couples the transmitting piece 30 and the jaw arrangement 18. Also the U-shaped cross-section of the retaining bracket 32 is shown in Fig. 6. In the mounted state, the receiving frame 16 and the retaining bracket 32 form a so-to-say closed frame which houses movable components, namely the transmitting piece 30, the first jaw 20 and the second jaw 22, and the slide pieces 68, 70 that are interposed therebetween.

Further, in a rear portion of the distal head assembly 14, connector cables 136 are arranged which are for instance housed in the support shaft 46 of the transmitting piece 30. The connector cables 136 are respectively connected to one of the first jaw 20 and the second jaw 22 as discussed hereinbefore. For instance, each of the connector cables 136 may be coupled with one of the slide pieces 68, 70, e.g. by soldering, so as to transmit power to the respective jaws 20, 22 that are coupled with the slide pieces 68, 70.

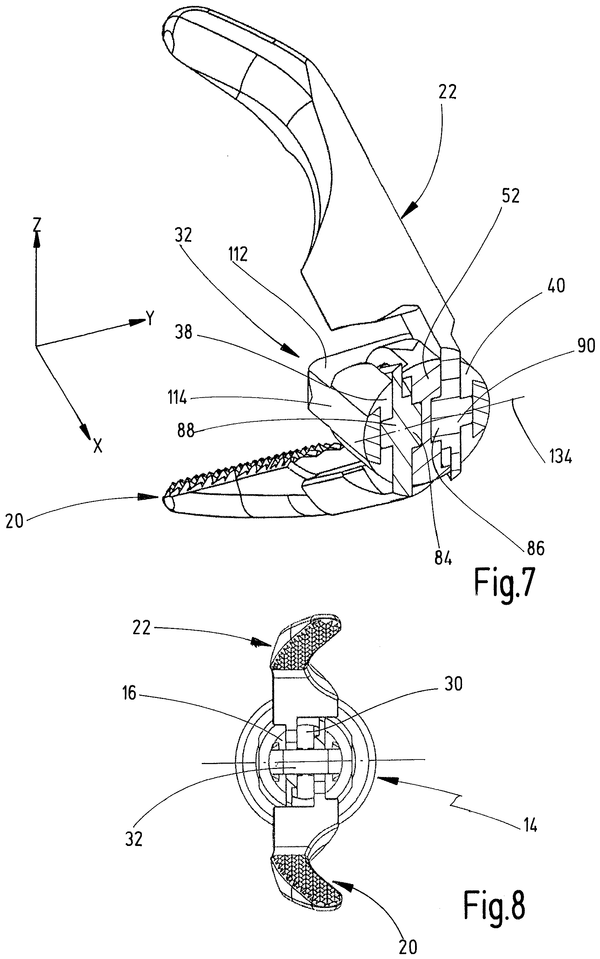

The perspective cross-sectional rear view of Fig. 7 illustrates the second, spaced-apart state of the jaws 20, 22 of the jaw arrangement 18. Fig. 8 is a supplemental frontal view of the arrangement of Fig. 7. A position of the cross-sectional plane of Fig. 7 is indicated in Fig. 6 by VII-VII. It is noted for the sake of completeness that Fig. 6 illustrates the first state and Fig. 7 illustrates the second state of the distal head assembly 14. However, the pivot axis 134 is maintained in both the first and the second state. Fig. 7 further illustrates a cross-sectional profile of the coupling arrangement jointly formed by the base grooves 118, 120 of the lateral arms 38, 40 of the receiving frame 16 and the retaining arms 114, 116 of the retaining bracket 32. The respective joints are arranged as dovetail joints. Hence, (laterally) inwardly or outwardly flexing of the lateral arms 38, 40 is prevented.

Preferably, outwardly facing lateral surfaces of the retaining arms 114, 116 are adapted to the basically rounded or cylindrical shape of the external surfaces of the lateral arms 38, 40. Hence, the distal head assembly 14 may exhibit a flush, integrated appearance. This may further facilitate operating, particularly moving an instrument 10 that is fitted with the head assembly 14. As can be further seen from Fig. 7, the movable components of the distal head assembly 14 are preferably arranged in a basically tight clearance fit fashion. This involves that only little (lateral) play is provided which improves accuracy. However, smooth running of the movable components is enabled by a remaining operating clearance. Hence, the retaining bracket 32 is not only provided for preventing the lateral arms 38, 40 from outwardly flexing, but also from inwardly flexing or from further inwardly-oriented distortions. Also shrinkage and similar manufacturing-related deformations may be compensated in this way. In other words, the retaining bracket 32 may be regarded as a lateral gage for the distal head assembly 14. The retaining bracket 32 ensures a parallel orientation of the first arm 38 and the second arm 40.

Fig. 8 further illustrates that the distal head assembly 14 provides a basically integrated and flush design. Preferably, neither component of the distal head assembly 14 substantially extends beyond the radial extension of the shaft 12 of the instrument 10. This applies in particular to the first, closed state of the jaw arrangement 18. Fig. 8 further illustrates that even in the second, open state of the jaw arrangement 18, neither component of the distal head assembly 12, particularly of the mechanical linkage 58 extends beyond the lateral extension of the shaft 12. Needless to say, the jaw arrangement 18 may also comprise specifically curved jaws that extend beyond the outline of the shaft 12, if required.

Components of the distal head assembly 14 that are not involved in the power transmission are preferably made from non-conductive materials. This applies in particular to the receiving frame 16, the transmitting piece 30 and preferably also to the retaining bracket 32. For instance non-conductive material such as polyether ether ketone (PEEK) or similar thermoplastics may be used. The jaws 20, 22 and the slide pieces 68, 70 can be made from conductive materials, at least in part, e.g. from metal-based materials.

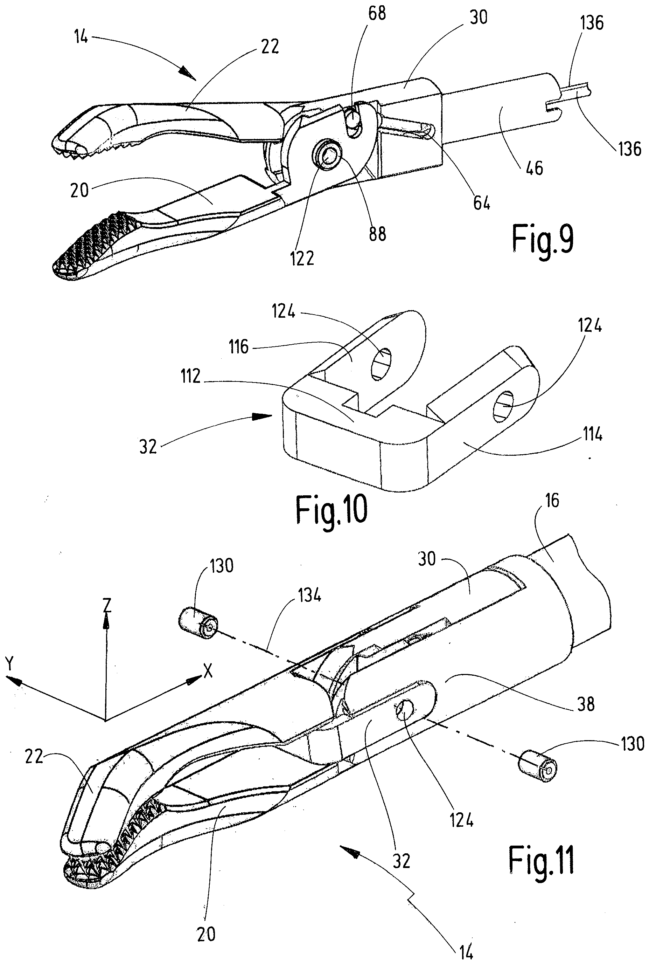

With particular reference to Figs. 9, 10 and 11, a further exemplary embodiment of a distal head assembly 14 for a bipolar instrument 10 in accordance with the present disclosure is described. As to the general layout of the distal head assembly 14, reference is made to the aspects and features explained hereinbefore in connection with Figs. 1 to 8.

Fig. 9 shows a sub-assembly of the distal head assembly 14, wherein the jaws 20, 22 engage the transmitting piece 30 and the slide pieces 68, 70 that are arranged in slide bases 64, 66 at the transmitting piece 30. Further, the jaws 20, 22 are provided with bearing elements 88, 90. At the bearing elements 88, 90, inner locking recesses 122 are provided that are arranged to receive locking pins 130 (refer to Fig. 11). As with the embodiment of Figs. 6 and 7, the bearing elements 88, 90 are arranged to extend, in the mounted state, through respective mounting recesses 100, 102 at lateral arms 38, 40 of the receiving frame 16. Hence, also the locking recesses 122 may extend through the mounting recesses 100, 102, and are basically laterally accessible. Preferably, the locking recesses 122 are aligned with a pivot axis 134 (refer also to Fig. 7 and to Fig. 11).

As can be seen in Fig. 10, also a respective retaining bracket 32 may be provided which includes a base 112 and retaining arms 114, 116 extending therefrom. At the retaining arms 114, 116, respective outer locking recesses 124 are provided. The locking recesses 124 are arranged as recesses or through holes. As already explained hereinbefore, the sub-assembly of Fig. 9 may be mounted to or inserted in a receiving portion 42 of the receiving frame 16. This may involve that the bearing elements 88, 90 engage the mounting recesses 100, 102 at the retaining bracket 32.

Further, as can be seen in Fig. 11, locking pins 130 may be provided which may secure or lock the mounted state of the retaining bracket 32. The locking pins 130 are arranged to engage the locking recesses 124 at the retaining bracket 32 and the inner locking recesses 122 at the bearing elements 88, 90. The bearing elements 88, 90, the mounting recesses 100, 102, the inner locking recesses 122, the outer locking recesses 124, and the locking pins 130 are arranged in concentric fashion and preferably aligned with the pivot axis 134. The locking pins 130 are arranged to connect the inner locking recesses 122 and the outer locking recesses 124 at respective sides of the distal head assembly 14. By way of example, the locking pins 130 extend through at least a portion of the inner locking recesses 122 and the outer locking recesses 124. The locking pins 130 may be bonded or otherwise fixed in a loss-proof fashion, e.g. in a force fit (tight fit) fashion.

Figs. 9 to 11 show an arrangement of the distal head assembly 14, wherein a first locking pin 130 is associated with a first lateral side, and wherein a second locking pin 130 is associated with a second lateral side of the distal head assembly 14. However, there may be alternative embodiments of the distal head assembly 14 that implement only a single locking pin 130 which is associated with one of the first or the second lateral side of the distal head assembly 14. Also a single locking pin 130 may reliably secure the mounted state of the retaining bracket 32.

Further reference is made to Fig. 12 illustrating an exemplary embodiment of a bipolar instrument 10 implementing a distal head assembly 14. As indicated above, the instrument 10 comprises an elongated shaft 12 which is illustrated in Fig. 12 in a broken state. At a distal end of the shaft 12, the distal head assembly 14 is provided. The distal head assembly 14 comprises a movable jaw arrangement 18. In accordance with at least some embodiments discussed herein, the movable jaw arrangement 14 comprises a first movable jaw 20 and a second movable jaw 22. The first jaw 20 is indicated in Fig. 12 by a dashed line representation. However, in some further exemplary embodiments, the distal head assembly 14 may comprise a first jaw 138 which is arranged as a stationary jaw. Further, a movable second jaw 22 may be provided. In practice, a relative pivoting movement between the first stationary jaw 138 and the second movable jaw 22 is provided. However, no relative pivoting movement between the first, stationary jaw 138 and the distal head assembly 14 or the shaft 12 of the instrument 10 is provided. Also a respective distal head assembly 14 comprising a stationary jaw 138 and a movable jaw 22 is encompassed by at least some aspects of the present disclosure.

At the proximal end of the shaft 12, the instrument 10 comprises a handling portion 140. For instance, the handling portion 140 may comprise a first handle 142 and a second handle 144 which may be moved relative to one another to effect the desired clamping action of the distal head assembly 14. Further, a connector housing 146 and respective lead connectors 148 may be provided. Via the lead connectors 148, the instrument 10 may be coupled to a respective control device and/or current supply means. Needless to say, also a common housing for the handle portion 140 and the connector housing 146 may be provided. In the exemplary embodiment of Fig. 12, the handles 142, 144 of the handling portion 140 are coupled to an actuating mechanism 150 which is connected with the distal head assembly 14 via the shaft 12. As a consequence, the jaw arrangement 14 may be moved between the first state and the second state when the handling portion 140 is actuated accordingly. However, this shall not be regarded as limiting the scope of the present disclosure. In the alternative, so-called motorized or powered bipolar instruments are known wherein a respective non-manual powered drive for the distal head assembly 14 is provided, for instance an electric motor, a magnetic actuator, and such like.

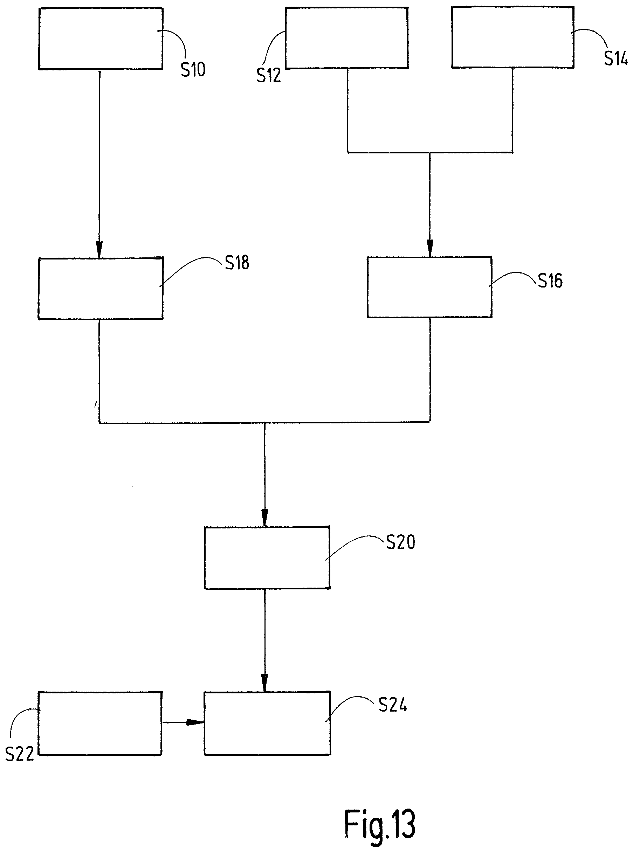

Further reference is made to Fig. 13 illustrating a simplified block diagram representing several steps of a method of assembling a distal head assembly for an instrument within the scope of the present disclosure.

In a step S10, a receiving frame is provided, the receiving frame comprising two lateral arms that are laterally spaced from one another and define therebetween a receiving portion. In a further step S12, a transmitting piece is provided. In a further step S14, a movable jaw arrangement is provided which may comprise a first jaw and a second jaw. At a step S14, the first jaw and the second jaw may be present as separate parts. In a sub-assembly step S16, the first jaw and the second jaw are attached to the transmitting piece at opposite sides thereof. The step S16 may further involve mounting slide pieces between the transmitting piece and the first jaw and the second jaw, respectively. In this way, major components of a mechanical linkage for driving the movable jaw arrangement may be assembled.

In a further step S18, at least one of the first lateral arm and the second lateral arm of the receiving frame is deflected in a defined fashion. In this way, a mounting gap or mounting space may be provided. A main assembly step S20 may follow wherein the sub-assembled transmitting piece and the jaw arrangement are attached to or inserted in a receiving portion between the first lateral arm and the second lateral arm of the receiving frame. Preferably, the step S20 involves a snap-on engagement of bearing elements respectively provided at the lateral arms of the receiving frame and the first and the second jaw. In this way, a pivoting axis for the jaw arrangement may be defined.

In a further step S22, a frontal retaining bracket is provided. The retaining bracket is arranged to secure the overall assembly arrangement. Therefore, an assembly securing step S24 may follow wherein the retaining bracket is attached to a frontal end of the receiving frame, particularly to the lateral arms thereof so as to secure the mounting state of the movable components of the distal head assembly, particularly of the jaw arrangement and the transmitting piece which is arranged between the first jaw and the second jaw of the jaw arrangement. Preferably, the retaining bracket is arranged to prevent outward and/or inward deflection of the first lateral arm and the second lateral arm, and to ensure a desired parallel orientation thereof.

The step S22 may optionally include a locking or securing (sub) step wherein at least one locking element is provided, preferably a locking pin, which may be laterally inserted and which may engage the retaining bracket and at least one of the receiving frame and the jaws in a loss-proof fashion. Hence, the retaining bracket may be prevented from being released, removed or "unplugged" from the receiving frame.

## Claims

1. A distal head assembly (14) for a bipolar instrument (10), the head assembly (14) comprising a receiving frame (16) comprising a first lateral arm (38) and a second lateral arm (40) that define therebetween a receiving portion (42), a transmitting piece (30) movably arranged at the receiving portion (42), a movable jaw arrangement (18) comprising a first jaw (20) and a second jaw (22), and a retaining bracket (32) arranged to engage the receiving frame (16), wherein the jaw arrangement (18) is pivotably mounted to the receiving frame (16) in a snap-on mounting fashion, and wherein the retaining bracket (32), in the mounted state, engages the receiving frame (16) and secures the mounted state of the jaw arrangement (18), **characterized in that** the retaining bracket (32) is arranged as a frontal locking bracket that is arranged to be attached to a frontal end (28) of the receiving frame (16) to secure the snap-on mounting of the jaw arrangement (18).

2. The assembly (14) as claimed in claim 1, wherein the jaw arrangement (18) comprises a first jaw (20) and a second jaw (22), wherein the first jaw (20) is mounted to the first lateral arm (38), wherein the second jaw (22) is mounted to the second lateral arm (40), wherein a mounting portion (52) of the transmitting piece (30) is interposed between the first jaw (20) and the second jaw (22), and wherein the jaw arrangement (18) is pivotably supported at the receiving frame (16) in a positive-locking fashion.

3. The assembly (14) as claimed in claim 1 or 2, wherein at least one of the first lateral arm (38) and the second lateral arm (40) is deflectable to allow the jaw arrangement (18) to engage the receiving portion (42) of the receiving frame (16), and wherein the retaining bracket (32), in the mounted state, retains the first lateral arm (38) and the second lateral arm (40) in a basically non-deflected state.

4. The assembly (14) as claimed in any of the preceding claims, wherein the retaining bracket (32) comprises a base (112), a first retaining arm (114) and a second retaining arm (116) extending therefrom in a basically longitudinal direction, wherein the first retaining arm (114) and the second retaining arm (116) are laterally spaced from one another, wherein the receiving frame (16) comprises a first base groove (118) arranged at the first lateral arm (38) and a second base groove (120) arranged at the second lateral arm (40), wherein the first base groove (118) and the first retaining arm (114) engage one another in the mounted state, wherein the second base groove (120) and the second retaining arm (116) engage one another in the mounted state, wherein the first base groove (118) and the first retaining arm (114) preferably define a longitudinally extending dovetail joint, and wherein the second base groove (120) and the second retaining arm (116) preferably define a longitudinally extending dovetail joint.

5. The assembly (14) as claimed in any of the preceding claims, wherein the jaw arrangement (18) comprises a first bearing element (88) and a second bearing element (90), the first bearing element (88) engaging the first lateral arm (38) and the second bearing element (90) engaging the second lateral arm (40), wherein the first bearing element (88) is arranged at the first jaw (20) to define a pivot axis thereof, and wherein the second bearing element (90) is arranged at the second jaw (22) to define a pivot axis thereof.

6. The assembly (14) as claimed in claim 5, wherein the first bearing element (88) is integrally formed at the first jaw (20) and engages a mounting recess (100) at the first lateral arm (38), and wherein the second bearing element (90) is integrally formed at the second jaw (22) and engages a mounting recess (102) at the second lateral arm (40).

7. The assembly (14) as claimed in any of the preceding claims, wherein the transmitting piece (30) comprises a first elongated slot (54) arranged at a first lateral side thereof and a second elongated slot (56) arranged at an opposite second lateral side thereof, wherein the first jaw (20) comprises a first mounting protrusion (84) engaging the first elongated slot (54), wherein the second jaw (22) comprises a second mounting protrusion (86) engaging the second elongated slot (56), wherein the first jaw (20) and the second jaw (22) are arranged to be inserted into the receiving portion (42), and wherein a main elongation direction of the first elongated slot (54) and the second elongated slot (56) is aligned with the longitudinal direction.

8. The assembly (14) as claimed in any of the preceding claims, wherein the first jaw (20) and the second jaw (22) are arranged at the receiving frame (16) and the transmitting piece (30) in a fashion pivotable with respect to one another between a first pivot state and a second pivot state about a laterally extending pivot axis defined by the receiving frame (16), wherein operating ends (24, 26) of the first jaw (20) and the second jaw (22) approach one another in the first pivot state and are spaced away from one another in the second pivot state, and wherein the first jaw (20) and the second jaw (22) jointly encompass the retaining bracket (32) in the first pivot state.

9. The assembly (14) as claimed in any of the preceding claims, further comprising a mechanical linkage (58) arranged to transfer a longitudinal movement of the transmitting piece (30) into a swiveling movement of the movable jaw arrangement (18), preferably a pivoting movement of the first jaw (20) and an the second jaw (22) in opposite direction, wherein the mechanical linkage (58) comprises two opposite slide joints (60, 62) including a first slide joint (60) that couples the transmitting piece (30) and the first jaw (20), and a second slide joint (62) that couples the transmitting piece (30) and the second jaw (22), and wherein the first slide joint (60) and the second slide joint (62) are oriented in an opposite inclination with respect to a longitudinal axis of the transmitting piece (30).

10. The assembly (14) as claimed in claim 9, wherein the first slide joint (60) comprises a first slide piece (68) movably arranged at a first slide base (64) of the transmitting piece (30), wherein the second slide joint (62) comprises a second slide piece (70) movably arranged at a second slide base (66) of the transmitting piece (30), wherein the first slide piece (68) engages a coupling portion of the first jaw (20) for driving the first jaw (20), and wherein the second slide piece (70) engages a coupling portion of the second jaw (22) for driving the second jaw (22).

11. The assembly (14) as claimed in claim 10, wherein the first slide base (64) is a slide groove arranged at the transmitting piece (30), wherein the second slide base (66) is a slide groove arranged at the transmitting piece (30) at an opposite side thereof, wherein the first slide piece (68) comprises a protruding engagement stud (72) engaging a mating coupling recess (92) at the coupling portion (80) of the first jaw (20), and wherein the second slide piece (70) comprises a protruding engagement stud (74) engaging a mating coupling recess (94) at the coupling portion (82) of the second jaw (22).

12. The assembly (14) as claimed in any of the preceding claims, wherein the first jaw (20) and the second jaw (22), particularly the operating ends (24, 26) thereof, are isolated from one another in a non-contact state of the operating ends (24, 26).

13. A bipolar instrument (10), particularly a coagulating instrument selected from the group consisting of bipolar forceps, bipolar scissors, a bipolar clamp and bipolar pincers, the instrument (10) comprising a distal head assembly (14) as claimed in any of the preceding claims.

14. A method of assembling the distal head assembly (14) according to any of claims 1-12 for a bipolar instrument (10), the method comprising the following steps:
- providing a receiving frame (16) comprising a first lateral arm (38) and a second lateral arm (40) that define therebetween a receiving portion (42),
- providing a transmitting piece (30) and a movable jaw arrangement (18) comprising a first jaw (20) and a second jaw (22),
- arranging the first jaw (20) and the second jaw (22) at the transmitting piece (30),
- inserting the sub-assembled transmitting piece (30) and the jaw arrangement (18) in the receiving portion (42) of the receiving frame (16) in a snap-on mounting fashion, comprising temporarily deflecting at least one of the first lateral arm (38) and the second lateral arm (40), wherein the first jaw (20) engages the transmitting piece (30) and the first lateral arm (38), and wherein the second jaw (22) engages the transmitting piece (30) and the second lateral arm (40), and
- securing the mounted state of the jaw arrangement (18) by attaching a retaining bracket (32) to the receiving frame (16).

15. The method as claimed in claim 14, wherein
the step of arranging the first jaw (20) and the second jaw (22) at the transmitting piece (30) further comprises
- mounting at least one slide piece (68, 70) in a slide groove (64, 66) at the transmitting piece (30),
- arranging at least one of the first jaw (20) and the second jaw (22) in a respective mounting orientation,
- approaching the transmitting piece (30) with the jaw (20, 22), including engaging the slide piece (68, 70) with a coupling recess (92, 94) of the jaw (20, 22) and an elongated slot (54, 56) arranged at a lateral side of the transmitting piece (30) with a mounting protrusion (84, 86) of the jaw (20, 22), and
the step of securing the mounted state of the jaw arrangement (18) by attaching a retaining bracket (32) to the receiving frame (16) further comprises
- attaching the retaining bracket (32) to a frontal end of the receiving frame (16), including engaging at least one base groove (118, 120) of the receiving frame (16) with at least one retaining arm (114, 116) of the retaining bracket (32), wherein the at least one base groove (118, 120) and the at least one retaining arm (114, 116) preferably form a longitudinally extending dovetail joint.

## Patentansprüche

1. Distale Kopfbaugruppe (14) für ein bipolares Instrument (10), wobei die Kopfbaugruppe (14) einen Aufnahmerahmen (16) umfasst, der einen ersten seitlichen Arm (38) und einen zweiten seitlichen Arm (40) umfasst, die zwischen sich einen Aufnahmeabschnitt (42) definieren, ein Übertragungsstück (30), das beweglich am Aufnahmeabschnitt (42) angeordnet ist, eine bewegliche Backenanordnung (18), die eine erste Backe (20) und eine zweite Backe (22) umfasst, und einen Haltebügel (32), der dazu ausgebildet ist, in den Aufnahmeabschnitt (16) einzugreifen, wobei die Backenanordnung (18) mittels einer Schnappbefestigung am Aufnahmerahmen (16) schwenkbar montiert ist, und wobei der Haltebügel (32) im montierten Zustand an den Aufnahmerahmen (16) angreift und den montierten Zustand der Backenanordnung (18) sichert, **dadurch gekennzeichnet, dass** der Haltebügel (32) als stirnseitiger Verriegelungsbügel angeordnet ist, der dazu ausgebildet ist, an einem stirnseitigen Ende (28) des Aufnahmerahmens (16) zur Sicherung der Schnappbefestigung der Backenanordnung (18) befestigt zu werden.

2. Baugruppe (14) nach Anspruch 1, wobei die Backenanordnung (18) eine erste Backe (20) und eine zweite Backe (22) umfasst, wobei die erste Backe (20) am ersten Seitenarm (38) montiert ist, wobei die zweite Backe (22) am zweiten Seitenarm (40) montiert ist, wobei ein Montageabschnitt (52) des Übertragungsstücks (30) zwischen der ersten Backe (20) und der zweiten Backe (22) angeordnet ist, und wobei die Backenanordnung (18) am Aufnahmerahmen (16) formschlüssig schwenkbar gelagert ist.

3. Baugruppe (14) nach Anspruch 1 oder 2, wobei mindestens einer des ersten Seitenarms (38) und des zweite Seitenarms (40) auslenkbar ist, so dass die Backenanordnung (18) an den Aufnahmeabschnitt (42) des Aufnahmerahmens (16) angreifen kann, und wobei der Haltebügel (32) im montierten Zustand den ersten Seitenarm (38) und den zweiten Seitenarm (40) in einem im Wesentlichen nicht abgelenkten Zustand hält.

4. Baugruppe (14) nach irgendeinem der vorhergehenden Ansprüche, wobei der Haltebügel (32) eine Basis (112), einen ersten Haltearm (114) und einen zweiten Haltearm (116) umfasst, die sich davon im Wesentlichen in einer Längsrichtung erstrecken, wobei der erste Haltearm (114) und der zweite Haltearm (116) seitlich voneinander beabstandet sind, wobei der Aufnahmerahmen (16) eine erste Basisnut (118), die am ersten seitlichen Arm (38) angeordnet ist, und eine zweite Basisnut (120) umfasst, die am zweiten seitlichen Arm (40) angeordnet ist, wobei die erste Basisnut (118) und der erste Haltearm (114) im montierten Zustand ineinander greifen, wobei die zweite Basisnut (120) und der zweite Haltearm (116) im montierten Zustand ineinander greifen, wobei die erste Basisnut (118) und der erste Haltearm (114) vorzugsweise eine sich in Längsrichtung erstreckende Schwalbenschwanzverbindung definieren, und wobei die zweite Basisnut (120) und der zweite Haltearm (116) vorzugsweise eine sich in Längsrichtung erstreckende Schwalbenschwanzverbindung definieren.

5. Baugruppe (14) nach irgendeinem der vorhergehenden Ansprüche, wobei die Backenanordnung (18) ein erstes Lagerelement (88) und ein zweites Lagerelement (90) umfasst, wobei das erste Lagerelement (88) mit dem ersten Seitenarm (38) und das zweite Lagerelement (90) mit dem zweiten Seitenarm (40) in Eingriff steht, wobei das erste Lagerelement (88) an der ersten Backe (20) angeordnet ist, um eine Drehachse der ersten Backe (20) zu definieren, und wobei das zweite Lagerelement (90) an der zweiten Backe (22) angeordnet ist, um eine Drehachse der zweiten Backe (22) zu definieren.

6. Baugruppe (14) nach Anspruch 5, wobei das erste Lagerelement (88) integral an der ersten Backe (20) ausgebildet ist und an eine Befestigungsaussparung (100) am ersten Seitenarm (38) angreift, und wobei das zweite Lagerelement (90) integral an der zweiten Backe (22) ausgebildet ist und an eine Befestigungsaussparung (102) am zweiten Seitenarm (40) angreift.

7. Baugruppe (14) nach irgendeinem der vorhergehenden Ansprüche, wobei das Übertragungsstück (30) einen ersten länglichen Schlitz (54), der an einer ersten seitlichen Seite davon angeordnet ist, und einen zweiten länglichen Schlitz (56) umfasst, der an einer gegenüberliegenden zweiten seitlichen Seite davon angeordnet ist, wobei die erste Backe (20) einen ersten Montagevorsprung (84) umfasst, der an den ersten länglichen Schlitz (54) angreift, wobei die zweite Backe (22) einen zweiten Montagevorsprung (86) umfasst, der an den zweiten länglichen Schlitz (56) angreift, wobei die erste Backe (20) und die zweite Backe (22) dazu ausgebildet sind, in den Aufnahmeabschnitt (42) eingeführt zu werden, und wobei eine Haupterstreckungsrichtung des ersten länglichen Schlitzes (54) und des zweiten länglichen Schlitzes (56) mit der Längsrichtung ausgerichtet ist.

8. Baugruppe (14) nach irgendeinem der vorhergehenden Ansprüche, wobei die erste Backe (20) und die zweite Backe (22) am Aufnahmerahmen (16) und das Übertragungsstück (30) derart angeordnet sind, dass sie zwischen einem ersten Schwenkzustand und einem zweiten Schwenkzustand um eine sich seitlich erstreckende Drehachse, die durch den Aufnahmerahmen (16) definiert ist, relativ zueinander verschwenkbar ist, wobei sich die Arbeitsenden (24, 26) der ersten Backe (20) und der zweiten Backe (22) im ersten Schwenkzustand einander angenähert und im zweiten Schwenkzustand voneinander beabstandet sind, und wobei die erste Backe (20) und die zweite Backe (22) gemeinsam im ersten Schwenkzustand den Haltebügel (32) umfassen.

9. Baugruppe (14) nach irgendeinem der vorhergehenden Ansprüche, ferner umfassend ein mechanisches Gestänge (58), das dazu ausgebildet ist, eine Längsbewegung des Übertragungsstücks (30) in eine Schwenkbewegung der beweglichen Backenanordnung (18) zu übertragen, vorzugsweise eine Schwenkbewegung der ersten Backe (20) und einer zweiten Backe (22) in entgegengesetzter Richtung, wobei das mechanische Gestänge (58) zwei gegenüberliegende Gleitgelenke (60, 62) umfasst, mit einem ersten Gleitgelenk (60), das das Übertragungsstück (30) und die erste Backe (20) koppelt, und einem zweiten Gleitgelenk (62), das das Übertragungsstück (30) und die zweite Backe (22) koppelt, und wobei das erste Gleitgelenk (60) und das zweite Gleitgelenk (62) mit einer gegenläufigen Neigung bezüglich einer Längsachse des Übertragungsstücks (30) ausgerichtet sind.

10. Baugruppe (14) nach Anspruch 9, wobei die erste Gleitverbindung (60) ein erstes Gleitstück (68) umfasst, das an einer ersten Gleitbasis (64) des Übertragungsstücks (30) beweglich angeordnet ist, wobei die zweite Gleitverbindung (62) ein zweites Gleitstück (70) umfasst, das an einer zweiten Gleitbasis (66) des Übertragungsstücks (30) beweglich angeordnet ist, wobei das erste Gleitstück (68) an einen Kupplungsabschnitt der ersten Backe (20) zum Antreiben der ersten Backe (20) angreift, und wobei das zweite Gleitstück (70) an einen Kupplungsabschnitt der zweiten Backe (22) zum Antreiben der zweiten Backe (22) angreift.

11. Baugruppe (14) nach Anspruch 10, wobei die erste Gleitbasis (64) eine am Übertragungsstück (30) angeordnete Gleitnut ist, wobei die zweite Gleitbasis (66) eine am Übertragungsstück (30) an einer dazu gegenüberliegenden Seite angeordnete Gleitnut ist, wobei das erste Gleitstück (68) einen vorstehenden Eingriffsbolzen (72) umfasst, der an eine angepasste Kupplungsaussparung (92) am Kupplungsabschnitt (80) der ersten Backe (20) angreift, und wobei das zweite Gleitstück (70) einen vorstehenden Eingriffsbolzen (74) umfasst, der an eine passende Kupplungsaussparung (94) am Kupplungsabschnitt (82) der zweiten Backe (22) angreift.

12. Baugruppe (14) nach irgendeinem der vorhergehenden Ansprüche, wobei die erste Backe (20) und die zweite Backe (22), insbesondere deren Arbeitsenden (24, 26), in einem Nicht-Kontakt-Zustand voneinander isoliert sind.

13. Bipolares Instrument (10), insbesondere Koagulationsinstrument, ausgewählt aus der Gruppe bestehend aus bipolaren Pinzetten, bipolaren Scheren, einer bipolaren Klemme und bipolaren Zangen, wobei das Instrument (10) eine distale Kopfbaugruppe (14) gemäß zumindest einem der vorhergehenden Ansprüche umfasst.

14. Verfahren zum Zusammenbau der distalen Kopfanordnung (14) nach einem der Ansprüche 1-12 für ein bipolares Instrument (10), wobei das Verfahren die folgenden Schritte umfasst:
- Bereitstellen eines Aufnahmerahmens (16), der einen ersten seitlichen Arm (38) und einen zweiten seitlichen Arm (40) umfasst, die zwischen sich einen Aufnahmeabschnitt (42) definieren,
- Bereitstellen eines Übertragungsstücks (30) und einer beweglichen Backenanordnung (18) mit einer ersten Backe (20) und einer zweiten Backe (22),
- Anordnen der ersten Backe (20) und der zweiten Backe (22) am Übertragungsstück (30),
- Einsetzen des Übertragungsstücks (30) und der Backenanordnung (18), die eine Unterbaugruppe bilden, in den Aufnahmeabschnitt (42) des Aufnahmerahmens (16) mittels einer Schnappbefestigung, umfassend das vorübergehende Umlenken mindestens eines des ersten Seitenarms (38) und des zweiten Seitenarms (40), wobei die erste Backe (20) an das Übertragungsstück (30) und den ersten Seitenarm (38) angreift und wobei die zweite Backe (22) an das Übertragungsstück (30) und den zweiten Seitenarm (40) angreift, und
- Sichern des montierten Zustands der Backenanordnung (18) durch Befestigen eines Haltebügels (32) am Aufnahmerahmen (16).

15. Verfahren nach Anspruch 14, wobei
der Schritt des Anordnens der ersten Backe (20) und der zweiten Backe (22) an dem Übertragungsstück (30) ferner umfasst
- Montieren zumindest eines Gleitstücks (68, 70) in einer Gleitnut (64, 66) am Übertragungsstück (30),
- Anordnen mindestens einer der ersten Backen (20) und der zweiten Backe (22) in einer jeweiligen Einbauorientierung,
- Annäherung an das Übertragungsstück (30) mit der Backe (20, 22), umfassend ein Angreifen an das Gleitstück (68, 70) mit einer Kupplungsaussparung (92, 94) der Backe (20, 22), und ein Angreifen an einen länglichen Schlitz (54, 56), der an einer seitlichen Seite des Übertragungsstücks (30) angeordnet ist, mit einem Befestigungsvorsprung (84, 86) der Backe (20, 22), und
der Schritt zum Sichern des montierten Zustands der Backenanordnung (18) durch Anbringen eines Haltebügels (32) an dem Aufnahmerahmen (16) ferner umfasst
- Befestigen des Haltebügels (32) an einem vorderen Ende des Aufnahmerahmens (16), umfassend ein Angreifen an mindestens eine Basisnut (118, 120) des Aufnahmerahmens (16) mit mindestens einem Haltearm (114, 116) des Haltebügels (32), wobei die mindestens eine Basisnut (118, 120) und der mindestens eine Haltearm (114, 116) vorzugsweise eine sich in Längsrichtung erstreckende Schwalbenschwanzverbindung bilden.

## Revendications

1. Ensemble de tête distale (14) pour un instrument bipolaire (10), l'ensemble de tête (14) comprenant un cadre de réception (16) comprenant un premier bras latéral (38) et un deuxième bras latéral (40) qui définissent entre eux une portion de réception (42), une pièce de transmission (30) disposée de manière mobile au niveau de la portion de réception (42), un agencement de mâchoires mobiles (18) comprenant une première mâchoire (20) et une deuxième mâchoire (22), et une console de retenue (32) disposée de manière à venir en prise avec le cadre de réception (16), l'agencement de mâchoires (18) étant monté de manière pivotante sur le cadre de réception (16) par un montage par encliquetage, et la console de retenue (32), dans l'état monté, venant en prise avec le cadre de réception (16) et fixant l'état monté de l'agencement de mâchoires (18), **caractérisé en ce que** la console de retenue (32) est prévue sous forme de console de verrouillage frontale qui est prévue pour être attachée à une extrémité frontale (28) du cadre de réception (16) pour fixer le montage par encliquetage de l'agencement de mâchoires (18).

2. Ensemble (14) selon la revendication 1, dans lequel l'agencement de mâchoires (18) comprend une première mâchoire (20) et une deuxième mâchoire (22), la première mâchoire (20) étant montée sur le premier bras latéral (38), la deuxième mâchoire (22) étant montée sur le deuxième bras latéral (40), une portion de montage (52) de la pièce de transmission (30) étant interposée entre la première mâchoire (20) et la deuxième mâchoire (22), et l'agencement de mâchoires (18) étant supporté de manière pivotante au niveau du cadre de réception (16) par un verrouillage par engagement positif.

3. Ensemble (14) selon la revendication 1 ou 2, dans lequel au moins l'un parmi le premier bras latéral (38) et le deuxième bras latéral (40) peut être dévié pour permettre à l'agencement de mâchoires (18) de venir en prise avec la portion de réception (42) du cadre de réception (16), et dans lequel la console de retenue (32), dans l'état monté, retient le premier bras latéral (38) et le deuxième bras latéral (40) dans un état essentiellement non dévié.

4. Ensemble (14) selon l'une quelconque des revendications précédentes, dans lequel la console de retenue (32) comprend une base (112), un premier bras de retenue (114) et un deuxième bras de retenue (116) s'étendant depuis celle-ci dans une direction essentiellement longitudinale, le premier bras de retenue (114) et le deuxième bras de retenue (116) étant espacés latéralement l'un de l'autre, le cadre de réception (16) comprenant une première gorge de base (118) disposée au niveau du premier bras latéral (38) et une deuxième gorge de base (120) disposée au niveau du deuxième bras latéral (40), la première gorge de base (118) et le premier bras de retenue (114) venant en prise l'un avec l'autre dans l'état monté, la deuxième gorge de base (120) et le deuxième bras de retenue (116) venant en prise l'un avec l'autre dans l'état monté, la première gorge de base (118) et le premier bras de retenue (114) définissant de préférence un joint en queue d'aronde s'étendant longitudinalement, et la deuxième gorge de base (120) et le deuxième bras de retenue (116) définissant de préférence un joint en queue d'aronde s'étendant longitudinalement.

5. Ensemble (14) selon l'une quelconque des revendications précédentes, dans lequel l'agencement de mâchoires (18) comprend un premier élément de palier (88) et un deuxième élément de palier (90), le premier élément de palier (88) venant en prise avec le premier bras latéral (38) et le deuxième élément de palier (90) venant en prise avec le deuxième bras latéral (40), le premier élément de palier (88) étant disposé au niveau de la première mâchoire (20) pour définir un axe de pivotement de celle-ci, et le deuxième élément de palier (90) étant disposé au niveau de la deuxième mâchoire (22) pour définir un axe de pivotement de celle-ci.

6. Ensemble (14) selon la revendication 5, dans lequel le premier élément de palier (88) est formé intégralement au niveau de la première mâchoire (20) et vient en prise avec un renfoncement de montage (100) au niveau du premier bras latéral (38), et dans lequel le deuxième élément de palier (90) est formé intégralement au niveau de la deuxième mâchoire (22) et vient en prise avec un renfoncement de montage (102) au niveau du deuxième bras latéral (40).

7. Ensemble (14) selon l'une quelconque des revendications précédentes, dans lequel la pièce de transmission (30) comprend une première fente allongée (54) disposée au niveau d'un premier côté latéral opposé de celle-ci et une deuxième fente allongée (56) disposée au niveau d'un deuxième côté latéral de celle-ci, la première mâchoire (20) comprenant une première saillie de montage (84) venant en prise avec la première fente allongée (54), la deuxième mâchoire (22) comprenant une deuxième saillie de montage (86) venant en prise avec la deuxième fente allongée (56), la première mâchoire (20) et la deuxième mâchoire (22) étant prévues pour être insérées dans la portion de réception (42), et une direction d'allongement principale de la première fente allongée (54) et de la deuxième fente allongée (56) étant alignée avec la direction longitudinale.

8. Ensemble (14) selon l'une quelconque des revendications précédentes, dans lequel la première mâchoire (20) et la deuxième mâchoire (22) sont disposées au niveau du cadre de réception (16) et de la pièce de transmission (30) de manière à pouvoir pivoter l'une par rapport à l'autre entre un premier état de pivotement et un deuxième état de pivotement autour d'un axe de pivotement s'étendant latéralement, défini par le cadre de réception (16), des extrémités fonctionnelles (24, 26) de la première mâchoire (20) et de la deuxième mâchoire (22) se rapprochant l'une de l'autre dans le premier état de pivotement et étant espacées l'une de l'autre dans le deuxième état de pivotement, et la première mâchoire (20) et la deuxième mâchoire (22) entourant ensemble la console de retenue (32) dans le premier état de pivotement.

9. Ensemble (14) selon l'une quelconque des revendications précédentes, comprenant en outre une liaison mécanique (58) prévue pour transférer un mouvement longitudinal de la pièce de transmission (30) en un mouvement de basculement de l'agencement de mâchoires mobiles (18), de préférence un mouvement de pivotement de la première mâchoire (20) et de la deuxième mâchoire (22) dans des directions opposées, la liaison mécanique (58) comprenant deux joints à coulisse opposés (60, 62) comportant un premier joint à coulisse (60) qui accouple la pièce de transmission (30) et la première mâchoire (20), et un deuxième joint à coulisse (62) qui accouple la pièce de transmission (30) et la deuxième mâchoire (22), et le premier joint à coulisse (60) et le deuxième joint à coulisse (62) étant orientés avec une inclinaison opposée par rapport à un axe longitudinal de la pièce de transmission (30).

10. Ensemble (14) selon la revendication 9, dans lequel le premier joint à coulisse (60) comprend une première pièce coulissante (68) disposée de manière mobile au niveau d'une première base de coulissement (64) de la pièce de transmission (30), le deuxième joint à coulisse (62) comprenant une deuxième pièce coulissante (70) disposée de manière mobile au niveau d'une deuxième base de coulissement (66) de la pièce de transmission (30), la première pièce coulissante (68) venant en prise avec une portion d'accouplement de la première mâchoire (20) pour entraîner la première mâchoire (20), et la deuxième pièce coulissante (70) venant en prise avec une portion d'accouplement de la deuxième mâchoire (22) pour entraîner la deuxième mâchoire (22).

11. Ensemble (14) selon la revendication 10, dans lequel la première base de coulissement (64) est une gorge de coulissement disposée au niveau de la pièce de transmission (30), la deuxième base de coulissement (66) étant une gorge de coulissement disposée au niveau de la pièce de transmission (30) au niveau d'un côté opposé de celle-ci, la première pièce coulissante (68) comprenant un ergot d'engagement saillant (72) venant en prise avec un renfoncement d'accouplement correspondant (92) au niveau de la portion d'accouplement (80) de la première mâchoire (20), et la deuxième pièce coulissante (70) comprenant un ergot d'engagement saillant (74) venant en prise avec un renfoncement d'accouplement correspondant (94) au niveau de la portion d'accouplement (82) de la deuxième mâchoire (22).

12. Ensemble (14) selon l'une quelconque des revendications précédentes, dans lequel la première mâchoire (20) et la deuxième mâchoire (22), en particulier les extrémités fonctionnelles (24, 26) de celles-ci, sont isolées l'une de l'autre dans un état non en contact des extrémités fonctionnelles (24, 26).

13. Instrument bipolaire (10), en particulier instrument de coagulation choisi parmi le groupe constitué de forceps bipolaires, de ciseaux bipolaires, d'un clamp bipolaire et d'une pince bipolaire, l'instrument (10) comprenant un ensemble de tête distale (14) selon l'une quelconque des revendications précédentes.

14. Procédé pour assembler l'ensemble de tête distale (14) selon l'une quelconque des revendications 1 à 12 pour un instrument bipolaire (10), le procédé comprenant les étapes suivantes :
- fourniture d'un cadre de réception (16) comprenant un premier bras latéral (38) et un deuxième bras latéral (40) qui définissent entre eux une portion de réception (42),
- fourniture d'une pièce de transmission (30) et d'un agencement de mâchoires mobiles (18) comprenant une première mâchoire (20) et une deuxième mâchoire (22),
- agencement de la première mâchoire (20) et de la deuxième mâchoire (22) au niveau de la pièce de transmission (30),
- insertion du sous-ensemble constitué de la pièce de transmission (30) et de l'agencement de mâchoires (18) dans la portion de réception (42) du cadre de réception (16) par montage par encliquetage, comprenant la déviation temporaire d'au moins l'un parmi le premier bras latéral (38) et le deuxième bras latéral (40), la première mâchoire (20) venant en prise avec la pièce de transmission (30) et le premier bras latéral (38), et la deuxième mâchoire (22) venant en prise avec la pièce de transmission (30) et le deuxième bras latéral (40), et
- fixation de l'état monté de l'agencement de mâchoires (18) en attachant une console de retenue (32) au cadre de réception (16).

15. Procédé selon la revendication 14, dans lequel
l'étape d'agencement de la première mâchoire (20) et de la deuxième mâchoire (22) au niveau de la pièce de transmission (30) comprend en outre
- le montage d'au moins une pièce coulissante (68, 70) dans une gorge de coulissement (64, 66) au niveau de la pièce de transmission (30),
- l'agencement d'au moins l'une parmi la première mâchoire (20) et la deuxième mâchoire (22) dans une orientation de montage respective,
- le rapprochement de la pièce de transmission (30) de la mâchoire (20, 22), y compris la mise en prise de la pièce coulissante (68, 70) avec un renfoncement d'accouplement (92, 94) de la mâchoire (20, 22) et d'une fente allongée (54, 56) disposée au niveau d'un côté latéral de la pièce de transmission (30) avec une saillie de montage (84, 86) de la mâchoire (20, 22), et
l'étape de fixation de l'état monté de l'agencement de mâchoires (18) en attachant une console de retenue (32) au cadre de réception (16) comprend en outre :
- la fixation de la console de retenue (32) à une extrémité frontale du cadre de réception (16), y compris la mise en prise d'au moins une gorge de base (118, 120) du cadre de réception (16) avec au moins un bras de retenue (114, 116) de la console de retenue (20), l'au moins une gorge de base (118, 120) et l'au moins un bras de retenue (114, 116) formant de préférence un joint en queue d'aronde s'étendant longitudinalement.
